(19) Europäisches Patentamt
European Patent Office
Office européen des brevets

(11) Veröffentlichungsnummer : **0 106 949**
**B1**

(12) # EUROPÄISCHE PATENTSCHRIFT

(45) Veröffentlichungstag der Patentschrift : **15.04.87**

(21) Anmeldenummer : **83107371.3**

(22) Anmeldetag : **27.07.83**

(51) Int. Cl.[4] : **C 07 D215/18**, C 07 D405/06, C 07 D405/12, C 07 D409/06, C 07 D409/12, C 07 D417/06, C 07 D417/12, C 07 D401/06, C 07 D401/12, A 01 N 43/42, A 01 N 43/48

(54) **3,7-Dichlor-8-chinolinderivate, Verfahren zu ihrer Herstellung und ihre Verwendung zur Bekämpfung unerwünschten Pflanzenwuchses.**

(30) Priorität : **05.08.82 DE 3229175**

(43) Veröffentlichungstag der Anmeldung : **02.05.84 Patentblatt 84/18**

(45) Bekanntmachung des Hinweises auf die Patenterteilung : **15.04.87 Patentblatt 87/16**

(84) Benannte Vertragsstaaten : **DE FR GB**

(56) Entgegenhaltungen :
**EP-A- 0 060 429**
**FR-A- 2 183 265**

(73) Patentinhaber : **BASF Aktiengesellschaft**
**Carl-Bosch-Strasse 38**
**D-6700 Ludwigshafen (DE)**

(72) Erfinder : **Hagen, Helmut, Dr.**
**Max-Slevogt-Strasse 17e**
**D-6710 Frankenthal (DE)**
Erfinder : **Kohler, rolf-Dieter, Dr.**
**Amselweg 3**
**D-6803 Edingen-Neckarhausen (DE)**
Erfinder : **Markert, Juergen, Dr.**
**Am Speyerweg 26**
**D-6704 Mutterstadt (DE)**
Erfinder : **Wuerzer, Bruno, Dr. Dipl.-Landwirt**
**Ruedigerstrasse 13**
**D-6701 Otterstadt (DE)**

**EP 0 106 949 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 3,7-Dichlor-8-chinolinderivate, Verfahren zu ihrer Herstellung, Herbizide, die diese Verbindungen als Wirkstoffe enthalten, und Verfahren zur Bekämpfung unerwünschten Pflanzenwuchses mit diesen Verbindungen.

Aus DE-OS 23 22 143, US-PS 2 661 276 und GB-PS 1 419 788 sind Chinolinderivate mit schwachen herbiziden Eigenschaften bekannt.

Es wurde gefunden, daß 3,7-Dichlor-8-chinolinderivate der Formel

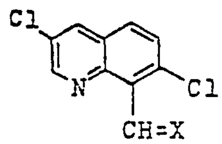

(I)

in der X für

a) =CHR oder

b) $=NR^5$ steht, wobei

R Wasserstoff, $C_1$- bis $C_8$-Alkyl, gegebenenfalls durch Phenyl substituiertes $C_2$- bis $C_6$-Alkenyl, $C_1$- bis $C_6$-Halogenalkyl, $C_2$- bis $C_6$-Halogenalkenyl, Cyano, einen gegebenenfalls durch einen oder mehrere Halogen-, Nitro-, Methyl-, Methoxy-, Carboxyreste substituierten Phenylring, gegebenenfalls substituiertes Furyl, Thienyl, Thiazolyl, Imidazolyl, Triazolyl, Pyridyl, Pyrimidinyl, Pyridazinyl, Pyrazinyl, Benzthiazolyl, Chinolinyl oder Benzoyl oder die Gruppen

$$-\underset{\underset{O}{\|}}{C}-NR^1R^2$$

und

$$-\underset{\underset{O}{\|}}{C}-OR^3$$

wobei $R^1$, $R^2$ und $R^3$ gleich oder verschieden sind und Wasserstoff, gegebenenfalls substituiertes $C_1$- bis $C_8$-Alkyl, gegebenenfalls substituiertes $C_2$- bis $C_6$-Alkenyl oder einen gegebenenfalls durch einen oder mehrere Halogen-, Nitro-, Methyl-, Methoxy-Carboxyreste substituierten Phenylring, bedeuten, und

$R^5$ Amino, Ureido, Thioureido, Cyclohexyl, Thiazolyl, Imidazolyl, Triazolyl, Pyrimidinyl, Pyridazinyl, Pyrazinyl, Thienyl, Furyl, Benzthiazolyl, Anilino,
wobei der Phenylrest durch eine oder mehrere Nitro-, Halogen-, Methyl-, Methoxy-, Hydroxy- oder Carboxygruppen substituiert sein kann, oder die Gruppe

$$O-\underset{\underset{O}{\|}}{C}-NH-R^6$$

wobei $R^6$ $C_1$- bis $C_8$-Alkyl, einen gegebenenfalls durch eine oder mehrere Nitro-, Halogen-, Methyl- oder Methoxygruppen substituierten Phenylring oder Cyclohexyl bedeutet, bedeuten:

Bemerkenswerte herbizide Wirkungen haben und dabei für Kulturpflanzen verträglich sind. So eignen sich die Verbindungen Ia) z. B. zur wirksamen Bekämpfung von Unkräutern der Gattungen Echinochloa und Galium. Die Verbindungen Ib) fallen wegen der Selektivität ihrer Wirkung auf. Bevorzugte Verbindungen der Formel I sind solche, bei denen $X=NR^5$ bedeutet.

In den neuen 3,7-Dichlor-8-chinolinderivaten, deren gemeinsames Merkmal die —CH= Gruppierung in 8-Stellung ist, können die gegebenenfalls durch Halogen substituierten Alkyl- und Alkenylreste geradkettig und, soweit es die Kohlenstoffzahl zuläßt, verzweigt sein. Bevorzugt sind solche Reste mit bis zu 4 Kohlenstoffatomen. Als Halogen sind Chlor und Brom vor Fluor bevorzugt. Beispielhaft sind zu nennen Methyl, Ethyl, Propyl, iso-Propyl, Butyl. Aryl ist in erster Linie Phenyl, außerdem auch Naphthyl.

Gegenstand der Erfindung sind weiterhin Verfahren sur Herstellung der 3,7-Dichlor-8-chinolinderivate.

Das eine Verfahren besteht darin, daß man ein 3,7-Dichlor-8-phosphoniummethylchinolin der Formel II mit Aldehyden der Formel III nach folgender Reaktionsgleichung umsetzt :

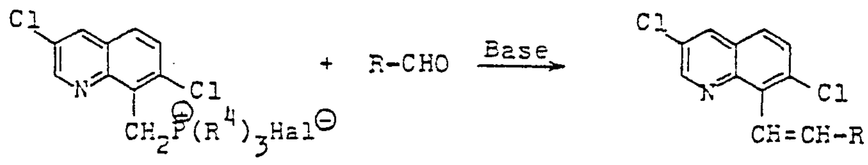


(II) (III) (Ia)

Dabei bedeutet $R^4$ Phenyl oder Alkyl und Hal Chlor, Brom oder Jod ; R hat die obengenannten Bedeutungen.

Ein anderes Verfahren zur Herstellung der 3,7-Dichlor-8-chinolinderivate der Formel Ia) besteht darin, daß man 3,7-Dichlor-8-formylchinolin der Formel IV mit Phosphoniumsalzen der Formel V nach folgender Reaktionsgleichung umsetzt :

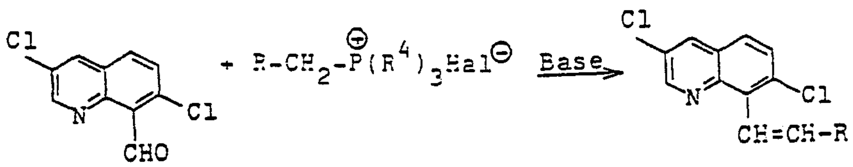


(IV) (V) (Ia) .

Dabei bedeutet $R^4$ Phenyl oder Alkyl, Hal Chlor, Brom oder Jod und R hat die obengenannten Bedeutungen.

Als Basen können z. B. verwendet werden : Natriumhydroxid, Kaliumhydroxid, Alkoholate, wie Natriummethylat, Natriumethylat, Kaliumethylat, Kalium-tert.-butylat, Amine, wie Pyridin, N-Methyl-morpholin, Natriumhydrid oder Natriumamid. Vorzugsweise wird Natriummethylat eingesetzt.

Die Umsetzungen werden zweckmäßigerweise in gegenüber den Reaktionspartnern inerten Lösungs- oder Verdünnungsmitteln durchgeführt. Hierfür sind beispielsweise geeignet :

Alkohole, wie Methanol, Ethanol, Propanole, Butanole ; Ether, wie Tetrahydrofuran, Dioxan, Diglykoldimethylether ; aromatische Kohlenwasserstoffe, wie Toluol, Xylole, Chlorbenzole ; Nitrile, wie Acetonitril, Propionitril ; Dimethylformamid ; Dimethylsulfoxid. Auch Gemische dieser Lösungs- und Verdünnungsmittel können verwendet werden.

Zur Durchführung der Verfahren setzt man die Ausgangsstoffe und Basen normalerweise in äquimolaren Mengen ein. Zweckmäßigerweise werden die Ausgangsstoffe im Lösungsmittel, z. B. in Ethanol, vorgelegt und die Base, z. B. Natriummethylat, zugegeben. Ein Überschuß der einen oder anderen Reaktionskomponente kann in einigen Fällen Vorteile bringen.

Die Reaktionstemperatur kann innerhalb eines größeren Bereichs variiert werden. Im allgemeinen arbeitet man bei Temperaturen im Bereich von 0 bis 100 °C, vorzugsweise im Bereich von 20 bis 50 °C.

Die Verbindungen der Formel Ib) können hergestellt werden, indem man den Aldehyd der Formel

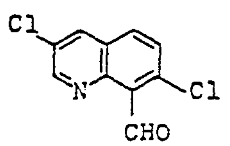

(VI)

mit einem Amin der Formel

$$H_2N—R^7 \qquad (VII)$$

umsetzt.

Darin bedeutet $R^7$ Amino, Benzthiazolyl, Ureido, Thioureido, Cyclohexyl, Thiazolyl, Imidazolyl, Triazolyl, Pyrimidinyl, Pyridazinyl, Pyrazinyl, Thienyl, Furyl und Anilino, wobei der Phenylrest gegebenenfalls durch eine oder mehrere Nitro-, Halogen-, Methyl- oder Methoxygruppen substituiert sein kann.

Die Umsetzung wird in dafür geeigneten Lösungsmitteln vorgenommen, wie Alkoholen, Dimethylformamid, Dimethylsulfoxid, Dioxan, und zwar bei Raumtemperatur bis 150 °C, vorzugsweise bei 80 °C bis 120 °C, innerhalb 1 bis 20 Stunden, vorzugsweise 2 bis 6 Stunden.

Die Verbindungen der Formel VI werden — soweit sie als Salz vorliegen — entweder vor der Reaktion z. B. mit Alkoholaten, Alkalihydroxiden, Triethylamin oder Carbonaten in ihre freien Basen überführt, oder als Salze zur Reaktion gebracht, wobei im Reaktionsmedium eine geeignete Base, wie Alkoholat,

Triethylamin, Hydroxid oder Carbonat, anwesend ist.

Die Oximcarbamate der Formel I, bei denen

$$X = N-O-\underset{\underset{O}{\|}}{C}-NHR^6$$

bedeutet, können hergestellt werden, indem man 3,7-Dichlor-8-chinolinaldoxim in an sich bekannter Weise mit Isocyanaten der Formel

$$O{=}C{=}N{-}R^6 \qquad \text{(VIII)}$$

in dafür geeigneten Lösungsmitteln, wie Dimethylformamid, Dimethylsulfoxid, Dioxan, Toluol, umsetzt. $R^6$ hat die oben angegebenen Bedeutungen.

Der Aldehyd der Formel IV wird erhalten, indem 3,7-Dichlor-8-dichlormethylchinolin in an sich bekannter Weise mit Schwefelsäure hydrolysiert wird. 3,7-Dichlor-8-dichlormethylchinolin wird aus 7-Chlor-8-methylchinolin oder -8-chlormethylchinolin oder -8-dichlormethylchinolin durch Chlorierung in Dichlorbenzol bei 150 bis 160 °C erhalten. Insbesondere kann diese Chlorierung in einem inerten Lösungsmittel, wie Dichlorbenzolen oder Trichlorbenzolen, in Anwesenheit eines Radikalstarters, wie Azoisobutyronitril oder Benzoylperoxid, unter Ausschluß von Licht, in einem Temperaturbereich von 140 bis 190 °C, vorzugsweise bei 150 bis 160 °C, durchgeführt werden.

Die folgenden Beispiele erläutern die Herstellung der Chinolinderivate der Formel I, dabei stellen die Verbindungen der Beispiele 26 und 27 Zwischenprodukte für die Herstellung der erfindungsgemäßen Verbindungen dar.

## Beispiel 1

3,7-Dichlor-8-[(1-methyl-5-nitro-imidazol-2-yl)-vinyl]-chinolin

36 ml einer 15 %igen Natriummethylat-Lösung in Methanol wurden bei 20 °C zu einer Mischung aus 50,6 g 3,7-Dichlor-8-triphenylphosphoniummethyl-chinolin-chlorid und 15,5 g 2-Formyl-1-methyl-5-nitro-imidazol getropft. Der gelbe Feststoff wurde nach 2 Stunden abgesaugt, mit Wasser gewaschen und getrocknet.

Ausbeute : 24 g (68 % d. Th.) ; Fp. 218 °C.

$C_{15}H_{10}Cl_2N_4O_2$ (349)

| | | | | | |
|---|---|---|---|---|---|
| Ber. : | C 51,6 | H 2,9 | Cl 20,3 | N 16,0 | O 9,2 |
| Gef. : | C 52,0 | H 3,1 | Cl 20,8 | N 15,4 | O 9,1 |

## Beispiel 2

1,2-Bis-(3,7-Dichlorchinolin-8-yl)-ethen

In die Suspension von 25,4 g 3,7-Dichlor-8-triphenylphosphoniummethyl-chinolin-chlorid und 11,3 g 3,7-Dichlor-8-formyl-chinolin in 150 ml Ethanol wurden bei Raumtemperatur 18 ml einer 15 %igen Natriummethylat-Lösung in Methanol getropft. Nach 2stündigem Rühren wurde der gelbe Niederschlag abgesaugt, mit Wasser behandelt und erneut abgesaugt.

Ausbeute : 20 g (95 d. Th.) ; Fp. 240 °C.

$C_{20}H_{10}Cl_4N_2$ (420)

| | | | | |
|---|---|---|---|---|
| Ber. : | C 57,1 | H 2,4 | Cl 33,8 | N 6,7 |
| Gef. : | C 57,1 | H 2,7 | Cl 33,5 | N 6,7 |

## Beispiel 3

3,7-Dichlor-8-vinyl-chinolin

25,4 g 3,7-Dichlor-8-triphenylphosphoniummethyl-chinolinchlorid und 5,5 g 30 %ige Formaldehydlösung in 150 ml Ethanol werden mit 18 ml einer 15 %igen Natriummethylatlösung in Methanol versetzt. Nach 1stündigem Rühren bei 20 °C wurde der Niederschlag abgesaugt, in Wasser aufgeschlämmt und erneut abgesaugt.

Ausbeute : 8 g (72 % d. Th.) ; Fp. 74 °C.

$C_{11}H_7Cl_2N$ (224)

Ber.: C 58,9 H 3,1 Cl 31,7 N 6,3
Gef.: C 57,8 H 3,4 Cl 32,9 N 5,6

## Beispiel 4

3,7-Dichlor-8-ethoxycarbonylvinyl-chinolin

Zu einer Suspension von 22,6 g 3,7-Dichlor-8-formylchinolin und 38,4 g Triphenylphosphoniumessigsäureethylester-chlorid in 200 ml Ethanol wurden bei 20 °C 36 ml einer 15 %igen Natriummethylat-Lösung in Methanol getropft. Nach 3stündigem Rühren wurde der Niederschlag abgesaugt, mit Wasser gewaschen und getrocknet.
Ausbeute: 18 g (60 % d. Th.); Fp. 116 °C.

$C_{14}H_{11}Cl_2NO_2$ (296)
Ber.: C 56,8 H 3,7 Cl 24,0 N 4,7 O 10,8
Gef.: C 55,9 H 3,6 Cl 24,4 N 4,9 O 11,4

## Beispiel 5

3,7-Dichlor-8-[2-(N-methyl-N-phenyl-carbamoyl)-vinyl]-chinolin

11,3 g 3,7-Dichlor-8-formyl-chinolin und 22,3 g Triphenylphosphoniumessigsäure-N-methylanilidchlorid in 150 ml Ethanol wurden mit 18 ml 15 %iges Natriummethylat in Methanol versetzt. Der gebildete Niederschlag wurde nach 4stündigem Rühren abgesaugt, mit Wasser gewaschen und getrocknet.
Ausbeute: 6 g (35 % d. Th.); Fp. 172 °C.

$C_{19}H_{14}Cl_2N_2O$ (357)
Ber.: C 63,9 H 3,9 Cl 19,9 N 7,8 O 4,5
Gef.: C 62,8 H 4,2 Cl 20,1 N 8,0 O 4,8

## Beispiel 6

3,7-Dichlor-8-[2-(2-chlorphenyl)-vinyl]-chinolin

25,4 g 3,7-Dichlor-8-triphenylphosphoniummethyl-chinolinchlorid und 7,7 g 2-Chlorbenzaldehyd wurden in 150 ml Ethanol gerührt und bei 20 °C mit 18 ml einer 15 %igen Natriummethylat-Lösung in Methanol versetzt. Der Niederschlag wird nach 6 Stunden abgesaugt, mit Wasser gewaschen und getrocknet.
Ausbeute: 15 g (89 % d. Th.); Fp. 114 °C.

$C_{17}H_{10}Cl_3N$ ( 334,5)
Ber.: C 61,0 H 3,0 Cl 31,8 N 4,2
Gef.: C 61,3 H 3,4 Cl 30,3 N 4,3

Auf analoge Weise wurden die in der folgenden Tabelle aufgeführten 3,7-Dichlor-8-chinolinderivate hergestellt.

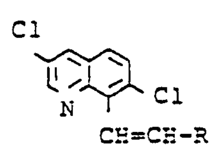

| Nr. | R | Fp. (°C) |
|---|---|---|
| 7 | 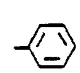 | 92 |
| 8 | 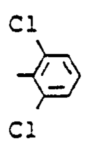 | 133 |

(Fortsetzung)

| Nr. | R | Fp. (°C) |
|---|---|---|
| 9 | 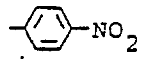 | 203 |
| 10 | 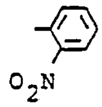 | 160 |
| 11 | 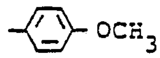 | 130 |
| 12 | 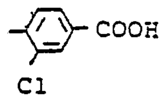 | > 220 |
| 13 | 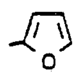 | 100 |
| 14 | 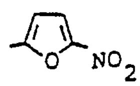 | 138 |
| 15 | 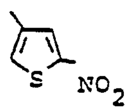 | 142 |
| 16 | 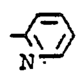 | 111 |
| 17 | 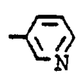 | 142 |
| 18 | 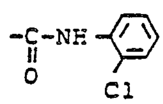 | 240 |
| 19 | 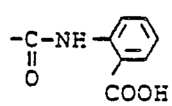 | > 260 |
| 20 | 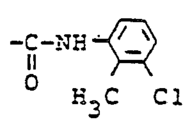 | > 260 |
| 21 | 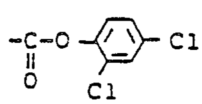 | 190 |
| 22 | $-COOH$ | > 260 |
| 23 | $-CN$ | 180 |

(Fortsetzung)

| Nr. | R | Fp. (°C) |
|---|---|---|
| 24 | 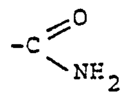 | 190 |
| 25 | 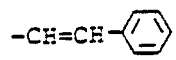 | 124 |

Beispiel 26

177 Teile 7-Chlor-8-methylchinolin und 1 Teil Azo-bisisobutyronitril werden in 1 000 Teilen Dichlorbenzol vorgelegt und auf 140 °C erhitzt. Bei dieser Temperatur wird mit dem Einleiten von 250 Teilen Chlor begonnen. Die Temperatur wird auf 160 °C erhöht. Nach erfolgter Reaktion wird die Lösung mit Stickstoff gespült, anschließend der größte Teil des Lösungsmittels abdestilliert, der ausgefallende Feststoff abgesaugt und mit Petrolether gewaschen. Man erhält 255 Teile 3,7-Dichlor-8-dichlormethylchinolin vom Fp. 154 °C.

Die Ausbeute entspricht 80 % d. Theorie.

Beispiel 27

56 Teile 3,7-Dichlor-8-dichlormethylchinolin werden in 250 Teilen 90 %iger Schwefelsäure 6 Stunden bei 100 °C gerührt. Die Lösung wird nach dem Abkühlen auf Eis gegossen, der ausgefallene Feststoff abgesaugt, mit Wasser neutral gewaschen und getrocknet. Man erhält 39 Teile 3,7-Dichlor-8-chinolincarbaldehyd vom Fp. 208 °C.

Die Ausbeute beträgt 87 % d. Theorie.

Beispiel 28

12 g Semicarbazidhydrochlorid und 5,8 g Natriummethylat werden in 300 ml Ethanol 10 Minuten zum Rückfluß erhitzt, abgekühlt und das ausgefallene Natriumchlorid abgesaugt. Zum Filtrat werden 23 g 3,7-Dichlor-8-chinolincarbaldehyd hinzugefügt und die Lösung 2 Stunden am Rückfluß gerührt. Man kühlt ab, versetzt mit Wasser, saugt das ausgefallene Produkt ab, wäscht mit Methanol nach und trocknet. Die Substanz wird aus Dimethylformamid umkristallisiert. Es werden 22 g (78 % d. Th.) 3,7-Dichlor-8-semicarbazonochinolin erhalten. Fp. > 280 °C.

Beispiel 29

23 g 3,7-Dichlor-8-chinolincarbaldehyd und 20 g 2,4-Dinitrophenylhydrazin werden in 500 ml Ethanol 5 Stunden auf 80 °C erhitzt. Das Reaktionsgemisch wird mit Wasser versetzt, der ausgefallene Feststoff abgesaugt und aus DMF umkristallisiert. Man erhält 34 g (85 % d. Th.) 3,7-Dichlor-8-(2,4-dinitrophenylhydrazono)-chinolin vom Fp. > 280 °C.

Beispiel 30

45 g 3,7-Dichlor-8-chinolincarbaldehyd und 10 g Hydraziniumhydroxid werden in 1 000 ml Ethanol suspendiert und 6 Stunden am Rückfluß erhitzt. Das Lösungsmittel wird abdestilliert, der schmierige Rückstand mit Ether versetzt, der Feststoff abgesaugt und aus Methoxipropanol umkristallisiert. Man erhält 37 g (78 % d. Th.) 3,7-Dichlor-8-chinolinalhydrazon vom Fp. 184 °C.

Beispiel 31

24 g 3,7-Dichlor-8-chinolinaldoxim werden in 250 ml Dimethylformamid gelöst, 12,5 g Cyclohexylisocyanat werden bei Raumtemperatur zugetropft und anschließend wird die Lösung 5 Stunden bei 50 °C gerührt. Innerhalb der Reaktionszeit werden nochmals 12 g Cyclohexylisocyanat langsam zugetropft. Die Reaktionslösung wird mit 2 l Wasser versetzt, der Feststoff abgesaugt, mit wenig Methanol aufgeschlämmt, wiederum abgesaugt und aus Toluol mit Aktivkohle umkristallisiert. Man erhält 26 g (72 % d. Th.) 3,7-Dichlor-8-chinolinaldoximcyclohexylcarbamat vom Fp. 152 °C.

Analog den Beispielen 1 bis 6 werden hergestellt :

| Bsp. | $R^5$ | Fp. (°C) |
|---|---|---|
| 32 | 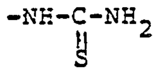 | |
| 33 | 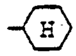 | 96 |
| 34 | 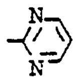 | |
| 35 | 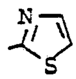 | |
| 36 | 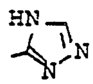 | 240 |
| 37 | 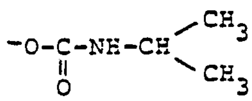 | 132 |
| 38 | 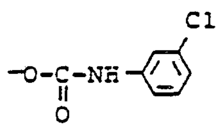 | 189 |
| 39 | 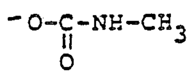 | 206 |
| 40 | 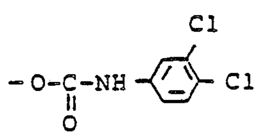 | - |
| 41 | 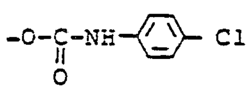 | - |
| 42 | 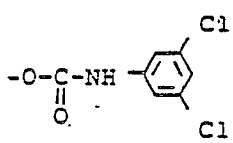 | - |
| 43 | 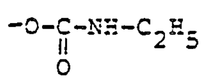 | 145 |
| 44 | 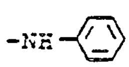 | 90 |

Die Applikation der neuen Verbindungen (Wirkstoffe) als herbizide Mittel kann im Vorauflaufverfahren und bei Nachauflaufanwendung erfolgen. Bei besonders empfindlichen Kulturpflanzen können Ausbringungstechniken angewandt werden, bei welchen die herbiziden Mittel mit Hilfe von Geräten so gespritzt werden, daß die Blätter der besonders empfindlichen Kulturpflanzen nach Möglichkeit nicht getroffen werden, während die Wirkstoffe auf die Blätter darunter wachsender unerwünschter Pflanzen oder auf die unbedeckte Bodenfläche gelagen (post-directed, lay-by).

Die Aufwandmengen an Wirkstoff betragen je nach Bekämpfungsziel, Jahreszeit, Zielpflanzen und Wachstumsstadien derselben ungefähr 0,1 bis 5 kg Wirkstoff/ha und mehr, vorzugsweise 1 bis 4 kg/ha. Im folgenden sei nur mit einigen Verbindungen beispielhaft gezeigt, welche vorzügliche Wirksamkeit und Selektivität sie haben.

Der Einfluß der 3,7-Dichlor-8-chinolinderivate auf das Wachstum von erwünschten und unerwünschten Pflanzen wird anhand von Gewächshausversuchen gezeigt :

Als Kulturgefäße dienten Plastikblumentöpfe mit 300 $cm^3$ Inhalt und lehmigem Sand mit etwa 1,5 % Humus als Substrat. Die Samen der Testpflanzen wurden nach Arten getrennt flach eingesät. Den Reis zog man in einem mit Torfmull (peat) angereicherten Substrat an, um ein günstigeres Wachstum zu gewährleisten. Unmittelbar danach erfolgt bei Vorauflaufbehandlung das Aufbringen der Wirkstoffe auf die Erdoberfläche. Sie wurden hierzu in Wasser als Verteilungsmittel suspendiert oder emulgiert und mittels fein verteilender Düsen gespritzt. Die Aufwandmengen betrugen im Vorauflauf 3,0 kg Wirkstoff/ha (Tabelle 1) und 1,0 oder 4,0 kg Wirkstoff/ha (Tabellen 4 bis 7). Nach dem Aufbringen der Mittel beregnete man die Gefäße leicht, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern diese nicht durch die Wirkstoffe beeinträchtigt wurden.

Für die Nachauflaufbehandlung zog man die Testpflanzen je nach Wuchsform erst bis zu einer Wuchshöhe von 3 bis 15 cm an und behandelte sie danach. Zur Nachauflaufbehandlung wurden entweder direkt gesäte und in den gleichen Gefäßen aufgewachsene Pflanzen ausgewählt oder aber sie wurden erst als Keimpflanzen getrennt angezogen und einige Tage vor der Behandlung in die Versuchsgefäße verpflanzt. Die Aufwandmenge für die Nachauflaufbehandlung betrug 3,0 kg Wirkstoff/ha (Tabellen 2 und 3) und 1,0 kg Wirkstoff/ha (Tabellen 4 und 5). Eine Abdeckung unterblieb bei der Nachauflaufbehandlung.

In die Versuche waren folgende Testpflanzen einbezogen :

| Botanischer Name | Deutscher Name |
|---|---|
| Avena sativa | Hafer |
| Beta vulgaris | Zuckerrübe |
| Brassica napus | Raps |
| Cassia tora | — |
| Echinochloa crus-galli | Hühnerhirse |
| Euphorbia geniculata | Südamerik. Wolfsmilch |
| Galium aparine | Klettenlabkraut |
| Oryza sativa | Reis |
| Sesbania exaltata | Turibaum |
| Setaria italica | Kolbenhirse |
| Solanum nigrum | Schwarzer Nachtschatten |
| Sorghum bicolor | Mohrenhirse (Kulturhirse) |
| Triticum aestivum | Weizen |

Die Versuchsgefäße wurden im Gewächshaus aufgestellt, wobei für wärmeliebende Arten wärmere Bereiche (20 bis 35 °C) und für solche gemäßigter Klimate 10 bis 20 °C bevorzugt wurden. Die Vergleichsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Aufgang und 100 kein Aufgang bzw. völlige Zerstörung zumindest der oberirdischen Sproßteile.

Bei der Vorauflaufanwendung von 3,0 kg Wirkstoff/ha zeigen z. B. die Verbindungen Nr. 3, 21 und 24 eine beachtliche herbizide Aktivität gegen das Beispielsgras Echinochloa crus-galli. Die Verbindungen Nr. 3 und 4 sind bei der gleichen Dosis im Nachauflaufverfahren wirksam gegen breitblättrige Pflanzen, wie Cassia tora.

Ebenso bekämpfen z. B. die Verbindungen Nr. 11, 13 und 16 mit 3,0 kg Wirkstoff/ha das Unkraut Galium aparine, ohne z. B. Hafer zu schädigen.

Gewächshausversuche zeigen weiterhin, daß die Verbindungen Nr. 36 und 44 mit 1,0 kg Wirkstoff/ha bei Vor- und Nachauflaufanwendung selektiv unerwünschte Pflanzen in Kulturpflanzenbeständen bekämpfen. Dasselbe vollbringt Verbindung Nr. 28 mit 1,0 kg/ha bei Vorauflaufanwendung. In diesen Versuchen bekämpft Wirkstoff Nr. 40 mit 4,0 kg Wirkstoff/ha bei Vorauflaufbehandlung beispielsweise auch breitblättrige Unkräuter in Zuckerrüben, ohne diese zu schädigen.

In Anbetracht der guten Verträglichkeit für zahlreiche breitblättrige und andere Kulturen und der Vielseitigkeit der Applikationsmethoden können die erfindungsgemäßen Herbizide oder diese enthaltende Mittel noch in einer weiteren großen Zahl von Kulturpflanzen zur Beseitigung unerwünschten Pflanzenwuchses eingesetzt werden.

In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor-Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glyoine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |

| Botanischer Name | Deutscher Name |
|---|---|
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung auch synergistischer Effekte können die neuen 3,7-Dichlor-8-chinolinderivate mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Tabelle 1

Herbizide Aktivität am Beispiel einer unerwünschten Grasart bei Vorauflaufanwendung im Gewächshaus

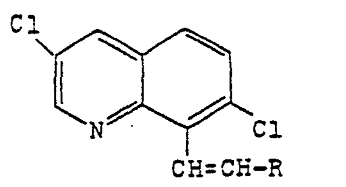

| Verbindung (Beispiel) Nr. | R | Schädigung der Testpflanze (%) bei 3,0 kg/ha Echinochloa crus-galli |
|---|---|---|
| 24 | 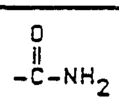 | 90 |
| 21 | 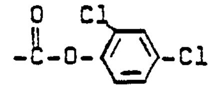 | 100 |
| 3 | H | 100 |

Tabelle 2

Herbizide Aktivität am Beispiel einer unerwünschten breitblättrigen Pflanze bei Nachauflaufanwendung im Gewächshaus

| Verbindung (Beispiel) Nr. | R | Schädigung der Testpflanze (%) bei 3,0 kg/ha Cassia tora |
|---|---|---|
| 3 | H | 100 |
| 4 | $-COOC_2H_5$ | 100 |

Tabelle 3

Selektive Bekämpfung von breitblättrigen Unkräutern in Getreide am Beispiel von Klettenlabkraut und Hafer bei Nachauflaufanwendung im Gewächshaus

| Verbindung (Beispiel) Nr. | R | Schädigung der Testpflanze (%) bei 3,0 kg/ha | |
|---|---|---|---|
| | | Avena sativa | Galium aparine |
| 11 | 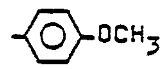 | 0 | 80 |
| 16 | 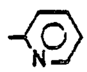 | 0 | 80 |
| 13 | 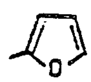 | 0 | 80 |

Tabelle 4

Selektive Bekämpfung von unerwünschtem Pflanzenwuchs mit Verbindung Nr 36 bei Vor- und Nachauflaufanwendung im Gewächshaus

| Testpflanzen | Schädigung (%) bei 1,0 kg/ha | |
|---|---|---|
| | Vorauflauf | Nachauflauf |
| Oryza sativa | 0 | 0 |
| Triticum aestivum | 0 | 0 |
| Setaria italica | 100 | 90 |
| Solanum nigrum | 95 | 95 |

Tabelle 5

Selektive Bekämpfung von breitblättrigen Unkräutern mit Verbindung Nr 44 bei Vor- und Nachauflaufanwendung im Gewächshaus

| Testpflanzen | Schädigung (%) bei 1,0 kg/ha | |
|---|---|---|
| | Vorauflauf | Nachauflauf |
| Brassica napus | 10 | 10 |
| Oryza sativa | 10 | 10 |
| Cassia tora | 98 | 100 |
| Euphorbia geniculata | 98 | 100 |
| Sesbania exaltata | 90 | 90 |
| Galium aparine | - | 95 |

Tabelle 6

Bekämpfung von breitblättrigen Unkräutern in verschiedenen Kulturen mit Verbindung Nr 28 bei Vorauflaufanwendung im Gewächshaus

| Testpflanzen | Schädigung (%) bei 1.0 kg/ha |
|---|---|
| Beta vulgaris | 0 |
| Brassica napus | 0 |
| Sorghum bicolor | 0 |
| Triticum aestivum | 0 |
| Euphorbia geniculata | 100 |
| Solanum nigrum | 98 |

Tabelle 7

Bekämpfung von unerwünschten breitblättrigen Pflanzen in Zuckerrüben mit Verbindung Nr 34 bei Vorauflaufanwendung im Gewächshaus

| Testpflanzen | Schädigung (%) bei 4,0 kg/ha |
|---|---|
| Beta vulgaris | 0 |
| Cassia tora | 100 |
| Euphorbia geniculata | 95 |
| Solanum nigrum | 95 |

**Patentansprüche**

1. 3,7-Dichlor-8-chinolinderivate der Formel

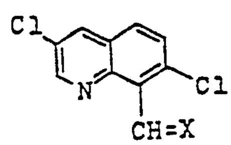

(I)

in der X für

a) =CHR oder

b) $=NR^5$ steht, wobei

R Wasserstoff, $C_1$- bis $C_8$-Alkyl, gegebenenfalls durch Phenyl substituiertes $C_2$- bis $C_6$-Alkenyl, $C_1$- bis $C_6$-Halogenalkyl, $C_2$- bis $C_6$-Halogenalkenyl, Cyano, ein gegebenenfalls durch einen oder mehrere Halogen-, Nitro-, Methyl-, Methoxy-, Carboxyreste substituierten Phenylring, gegebenenfalls substituiertes Furyl, Thienyl, Thiazolyl, Imidazolyl, Triazolyl, Pyridyl, Pyrimidinyl, Pyridazinyl, Pyrazinyl, Benzthiazolyl, Chinolinyl, Benzoyl oder die Gruppen

$$-\underset{\underset{O}{\|}}{C}-NR^1R^2$$

und

$$-\underset{\underset{O}{\|}}{C}-OR^3$$

wobei $R^1$, $R^2$ und $R^3$ gleich oder verschieden sind und Wasserstoff, gegebenenfalls substituiertes $C_1$- bis $C_8$-Alkyl, gegebenenfalls substituiertes $C_2$- bis $C_6$-Alkenyl oder einen gegebenenfalls durch einen oder

mehrere Halogen-, Nitro-, Methyl-, Methoxy- oder Carboxyreste substituierten Phenylring bedeuten, und

$R^5$ Amino, Ureido, Thioureido, Cyclohexyl, Thiazolyl, Imidazolyl, Triazolyl, Pyrimidinyl, Pyridazinyl, Pyrazinyl, Thienyl, Furyl, Benzthiazolyl, Anilino,

wobei der Phenylrest durch eine oder mehrere Nitro-, Halogen-, Methyl-, Methoxy-, Hydroxy- oder Carboxygruppen substituiert sein kann, oder die Gruppe

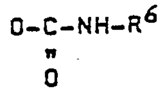

wobei $R^6$ $C_1$- bis $C_8$-Alkyl, einen gegebenenfalls durch eine oder mehrere Nitro-, Halogen-, Methyl- oder Carboxygruppen substituierten Phenylring oder Cyclohexyl bedeutet, bedeuten.

2. 3,7-Dichlor-8-chinolin-derivate der Formel I, dadurch gekennzeichnet, daß $X=NR^5$ bedeutet.

3. Verfahren zur Herstellung von 3,7-Dichlor-8-chinolinderivaten der Formel Ia) gemäß Anspruch 1, dadurch gekennzeichnet, daß man Phosphoniummethylchinoline der Formel II

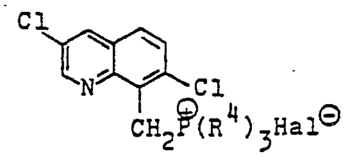

(II)

in der $R^4$ für Phenyl oder Alkyl steht und Hal Chlor, Brom oder Jod bedeutet, mit Aldehyden der Formel

$$R—CHO \qquad (III)$$

in der R die in Anspruch 1 genannten Bedeutungen hat, umsetzt.

4. Verfahren zur Herstellung von 3,7-Dichlor-8-chinolinderivaten der Formel Ia) gemäß Anspruch 1, dadurch gekennzeichnet, daß man 3,7-Dichlor-8-formylchinolin mit Phosphoniumsalzen der Formel

$$R-CH_2\overset{\oplus}{P}(R^4)_3 \quad Hal^{\ominus} \qquad (V)$$

in der R die in Anspruch 1 genannten Bedeutungen hat, $R^4$ für Phenyl oder Alkyl steht und Hal Chlor, Brom oder Jod bedeutet, umsetzt.

5. Verfahren zur Herstellung von 3,7-Dichlor-8-chinolinderivaten der Formel Ib) gemäß Anspruch 1, dadurch gekennzeichnet, daß man 3,7-Dichlor-8-formyl-chinolin mit einem Amin der Formel

$$H_2N—R^7 \qquad (VI)$$

in der $R^7$ Amino, Benzthiazolyl, Ureido, Thioureido, Cyclohexyl, Thiazolyl, Imidazolyl, Triazolyl, Pyrimidinyl, Pyridazinyl, Pyrazinyl, Thienyl, Furyl oder Anilino, wobei der Phenylrest gegebenenfalls durch eine oder mehrere Nitro-, Halogen-, Methyl- oder Methoxygruppen substituiert sein kann, bedeutet, umsetzt.

6. Verfahren zur Herstellung von 3,7-Dichlor-8-chinolinderivaten der Formel Ib) gemäß Anspruch 1, dadurch gekennzeichnet, daß man 3,7-Dichlor-8-chinolinaldoxim mit Isocyanaten der Formel

$$O=C=N—R^6 \qquad (VII)$$

in der $R^6$ die in Anspruch 1 genannten Bedeutungen hat, umsetzt.

7. Herbizid, enthaltend eine Verbindung der Formel I gemäß Anspruch 1.

8. Herbizid, enthaltend eine Verbindung der Formel I gemäß Anspruch 1 und inerte Zusatzstoffe.

9. Verfahren zur Herstellung von Herbiziden, dadurch gekennzeichnet, daß man mindestens eine Verbindung der Formel I gemäß Anspruch 1 mit inerten Zusatzstoffen mischt.

10. Verfahren zur Bekämpfung unerwünschten Pflanzenwuchses, dadurch gekennzeichnet, daß man die Pflanzen und/oder den Boden mit einer herbizid wirksamen Menge einer Verbindung der Formel I gemäß Anspruch 1 behandelt.

**Claims**

1. A 3,7-dichloro-8-quinoline derivative of the formula

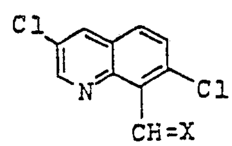

(I)

where X is
a) =CHR or
b) $=NR^5$, where

R is hydrogen, $C_1$-$C_8$-alkyl, unsubstituted or phenyl-substituted $C_2$-$C_6$-alkenyl, $C_1$-$C_6$-haloalkyl, $C_2$-$C_6$-haloalkenyl, cyano, or phenyl which is unsubstituted or substituted by one or more halogen atoms or one or more nitro, methyl, methoxy or carboxyl groups, or is unsubstituted or substituted furyl, thienyl, thiazolyl, imidazolyl, triazolyl, pyridyl, pyrimidinyl, pyridazinyl, pyrazinyl, benzothiazolyl, quinolyl or benzoyl, or the group

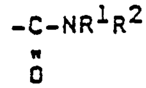

or

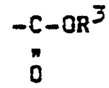

where $R^1$, $R^2$ and $R^3$ are identical or different and are each hydrogen, unsubstituted or substituted $C_1$-$C_8$-alkyl, unsubstituted or substituted $C_2$-$C_6$-alkenyl, or phenyl which is unsubstituted or substituted by one or more halogen atoms or one or more nitro, methyl, methoxy or carboxyl groups, and

$R^5$ is amino, ureido, thioureido, cyclohexyl, thiazolyl, imidazolyl, triazolyl, pyrimidinyl, pyridazinyl, pyrazinyl, thienyl, furyl benzothiazolyl or anilino,
the phenyl radical being optionally substituted by one or more halogen atoms or one or more nitro, methyl, methoxy, hydroxyl or carboxyl groups, or $R^5$ is

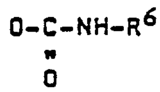

where $R^6$ is $C_1$-$C_8$-alkyl or is phenyl which is unsubstituted or substituted by one or more halogen atoms or one or more nitro, methyl or carboxyl groups, or is cyclohexyl.

2. A 3,7-dichloro-8-quinoline derivative of the formula I, where X is $=N-R^5$.

3. A process for the preparation of 3,7-dichloro-8-quinoline derivatives of the formula Ia) as claimed in claim 1, wherein a phosphonium methylquinoline of the formula II

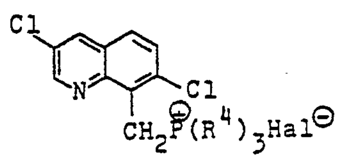

(II)

where $R^4$ is phenyl or alkyl and Hal is chlorine, bromine or iodine, is reacted with an aldehyde of the formula

$$R-CHO \qquad III,$$

where R has the meanings given in claim 1.

4. A process for the preparation of 3,7-dichloro-8-quinoline derivatives of the formula Ia) as claimed in claim 1, wherein a 3,7-dichloro-8-formylquinoline is reacted with a phosphonium salt of the formula

$$R-CH_2\overset{\oplus}{P}(R^4)_3 \quad Hal^{\ominus} \qquad (V)$$

where R has the meanings given in claim 1, $R^4$ is phenyl or alkyl, and Hal is chlorine, bromine or iodine.

5. A process for the preparation of 3,7-dichloro-8-quinoline derivatives of the formula Ib) as claimed in claim 1, wherein a 3,7-dichloro-8-formylquinoline is reacted with an amine of the formula

$$H_2N-R^7 \qquad (VI),$$

where $R^7$ is amino, benzothiazolyl, ureido, thioureido, cyclohexyl, thiazolyl, imidazolyl, triazolyl, pyrimidinyl, pyridazinyl, pyrazinyl, thienyl, furyl or anilino, and the phenyl radical can be unsubstituted or substituted by one or more halogen atoms or one or more nitro, methyl or methoxy groups.

6. A process for the manufacture of 3,7-dichloro-8-quinoline derivatives of the formula Ib) as claimed in claim 1, wherein a 3,7-dichloroquinoline-8-aldoxime is reacted with an isocyanate of the formula

O=C=N—$R^6$ (VII),

where $R^6$ has the meanings given in claim 1.

7. A herbicide containing a compound of the formula I as claimed in claim 1.

8. A herbicide containing a compound of the formula I as claimed in claim 1, an inert additives.

9. A process for the production of herbicides, wherein at least one compound of the formula I as claimed in claim 1 is mixed with inert additives.

10. A process for combating the growth of unwanted plants, wherein the plants and/or the soil are treated with a herbicidally effective amount of a compound of the formula I as claimed in claim 1.

**Revendications**

1. Dérivés de 3,7-dichloro-8-quinoline de formule

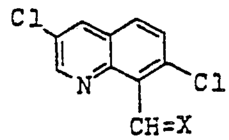

(I)

dans laquelle X est mis pour

a) =CHR ou

b) =$NR^5$

R représentant hydrogène, alkyle en $C_1$ à $C_8$, alcényle en $C_2$ à $C_6$, halogénalkyle en $C_1$ à $C_6$, halogénalcényle en $C_2$ à $C_6$, éventuellement substitués par phényle, cyano, un noyau phényle, éventuellement substitué par un ou plusieurs restes halogène, nitro, méthyle, méthoxy, carboxy, furyle, thiényle, thiazolyle, imidazolyle, triazolyle, pyridyle, pyrimidinyle, pyridazinyle, pyrazinyle, benzothiazolyle, quinolinyle, benzoyle éventuellement substitué ou les groupes

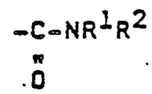

et

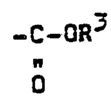

$R^1$, $R^2$ et $R^3$ étant identiques ou différents et représentant hydrogène, alkyle en $C_1$ à $C_8$, éventuellement substitué, alcényle en $C_2$ à $C_6$, éventuellement substitué ou un noyau phényle, éventuellement substitué par un ou plusieurs restes halogène, nitro, méthyle, méthoxy ou carboxy, et

$R^5$ représentant amino, uréido, thiouréido, cyclohexyle, thiazolyle, imidazolyle, triazolyle, pyrimidinyle, pyridazinyle, pyrazinyle, thiényle, furyle, benzothiazolyle, anilino,

le reste phényle pouvant être substitué par un ou plusieurs groupes nitro, halogène, méthyle, méthoxy, hydroxy ou carboxy, ou le groupe

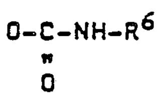

$R^6$ représentant alkyle en $C_1$ à $C_8$, un noyau phényle, éventuellement substitué par un ou plusieurs groupes nitro, halogène, méthyle ou carboxy, ou cyclohexyle.

2. Dérivés de 3,7-dichloro-8-quinoline de formule I, caractérisés par le fait que X représente =$NR^5$.

3. Procédé de préparation de dérivés de 3,7-dichloro-8-quinoline de formule Ia) selon la revendication 1, caractérisé par le fait que l'on fait réagir une phosphonium-méthylquinoline de formule II

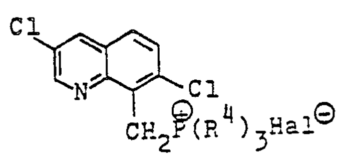

(II)

dans laquelle $R^4$ est mis pour phényle ou alkyle et Hal représente chlore, brome ou iode, avec des aldéhydes de formule

R—CHO (III),

dans laquelle R a les significations indiquées dans la revendication 1.

4. Procédé de préparation de dérivés de 3,7-dichloro-8-quinoline de formule Ia) selon la revendication 1, caractérisé par le fait que l'on fait réagir de la 3,7-dichloro-8-formylquinoline avec des sels de phosphonium de formule

$$R\text{-}CH_2\overset{\oplus}{P}(R^4)_3 \qquad Hal^{\ominus} \tag{V}$$

dans laquelle R a les significations données dans la revendication 1, $R^4$ représente phényle ou alkyle et Hal représente chlore, brome ou iode.

5. Procédé de préparation de dérivés de 3,7-dichloro-8-quinoline de formule Ib) selon la revendication 1, caractérisé par le fait que l'on fait réagir de la 3,7-dichloro-8-formylquinoline avec une amine de formule

$$H_2N\text{—}R^7 \qquad \text{(VI),}$$

dans laquelle $R^7$ représente amino, benzothiazolyle, uréido, thiouréido, cyclohexyle, thiazolyle, imidazolyle, pyrimidinyle, pyridazinyle, pyrazinyle, thiényle, furyle ou anilino, le reste phényle pouvant être substitué par un ou plusieurs groupes nitro, halogène, méthyle ou méthoxy.

6. Procédé de préparation de dérivés de 3,7-dichloro-8-quinoline de formule Ib) selon la revendication 1, caractérisé par le fait que l'on fait réagir de la 3,7-dichloro-8-quinolinaldoxime avec des isocyanates de formule

$$O{=}C{=}N\text{—}R^6 \tag{VII}$$

dans laquelle $R^6$ a les significations indiquées dans la revendication 1.

7. Herbicide, contenant un composé de formule I selon la revendication 1.

8. Herbicide, contenant un composé de formule I selon la revendication 1 et des additifs inertes.

9. Procédé de préparation d'herbicides, caractérisé par le fait que l'on mélange au moins un composé de formule I selon la revendication 1 avec des additifs inertes.

10. Procédé pour lutter contre la croissance de plantes indésirables, caractérisé par le fait que l'on traite les plantes et/ou le sol avec une quantité efficace, au point de vue herbicide, d'un composé de formule I selon la revendication 1.